(19) Europäisches Patentamt
European Patent Office
Office européen des brevets

(11) **EP 3 326 631 B1**

(12) **EUROPEAN PATENT SPECIFICATION**

(45) Date of publication and mention of the grant of the patent:
**14.07.2021 Bulletin 2021/28**

(21) Application number: **15898669.5**

(22) Date of filing: **23.07.2015**

(51) Int Cl.:
***A61K 31/495*** *(2006.01)*
***A61K 9/20*** *(2006.01)*
***A61K 9/48*** *(2006.01)*
***A61P 35/04*** *(2006.01)*
***A61P 35/00*** *(2006.01)*
***C07D 295/15*** *(2006.01)*

(86) International application number:
**PCT/CN2015/084912**

(87) International publication number:
**WO 2017/012113 (26.01.2017 Gazette 2017/04)**

(54) **COMPOUND PAC-1 OR SALT THEREOF, AND PHARMACEUTICAL COMPOSITION COMPRISING SAME**

PAC-1-VERBINDUNG ODER SALZ DAVON SOWIE PHARMAZEUTISCHE ZUSAMMENSETZUNGEN DAMIT

COMPOSÉ DE PAC -1 OU SEL DE CELUI-CI, ET COMPOSITION PHARMACEUTIQUE COMPRENANT CELUI-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**30.05.2018 Bulletin 2018/22**

(73) Proprietor: **Wang, Zhimin**
**Beijing 100700 (CN)**

(72) Inventor: **Wang, Zhimin**
**Beijing 100700 (CN)**

(74) Representative: **Maiwald Patent- und Rechtsanwaltsgesellschaft mbH**
**Elisenhof**
**Elisenstraße 3**
**80335 München (DE)**

(56) References cited:
**WO-A1-2010/091382**
**WO-A2-2006/128173**
**WO-A2-2008/134474**
**CN-A- 104 910 100**
**CN-A- 105 348 222**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 326 631 B1

**Description**

FIELD OF THE INVENTION

The present invention belongs to the technical field of medicinal chemistry, in particular to a method of preparing ZYS-1 (PAC-1 in amorphous form), a pharmaceutical composition containing the same, and a pharmaceutical use thereof.

BACKGROUND OF THE INVENTION

Tumor is the number one killer endangering human health, wherein the mortality rate by malignant tumors ranks first compared to other diseases. Since the middle of the 20th century, human beings have invested a great deal of efforts and material resources in the prevention and treatment of cancer. However, their harm has not yet been effectively controlled and their morbidity and mortality rate are still increasing year by year. At present the three major means of cancer treatment are chemotherapy, radiotherapy and surgical treatment, of which chemotherapy is the main approach. With the continuous development and the rational application of anti-cancer drugs, chemotherapy playsan increasingly important rolein the treatment of malignant tumors, and presentsa development trend of from the palliative to curative treatment. At present, commonly used anti-tumor drugs include cytotoxic drugs, hormonal drugs, molecular targeted therapies, biological response modifiers, tumor differentiation inducing agents, tumor angiogenesis inhibitors and drugs that assist in the treatment of tumors. The most common used one is the cytotoxic drug. Although it is highly efficient, there arc also toxic side effects, lack of selectivity and other shortcomings. In recent years, with the in-depth study of tumor characteristics and essence, anti-cancer drug therapy is developing from the traditionally cytotoxic drug treatment to the ones targeting cell receptors, key genes and regulatory molecules. Clinical efficacy indicators for anti-cancer drugs also transform from the pursuit of substantial reduction of tumor body to extending the survival of patients and/or improve the life quality. The PAC-1 is in line with this trend for developing small-molecule targetedanti-tumor drugs.

PAC-1 is the first small-molecule compound that directly activates procaspase-3, which was discovered by Quinn P. Peterson from the University of Illinois in the in vitro screening of more than 20,000 compounds. The compoundis named as PAC-1, whose chemical name is 1-Piperazineacetic acid, 4-(phenylmethyl)-2- [[2-hydroxy- 3-(2-propen- 1-yl) phenyl] methylene], and its structure is as shown in formula (I). Studies have shown that, PAC-1 can selectively induce apoptosis of tumor cells, and its induction of apoptosis is positively correlated with the level ofintracellular procaspasse-3, whereas the normal cells have a very low level of procaspasse-3 and are less likely to be killed by PAC-1, suggesting good selectivity of PAC-1. Its mechanism of action has been studied to show that procaspasse-3 autonomously activates into caspase-3 and induces apoptosis mainly by chelation inhibition of zinc ions (Quinn P, et al., J Mol Biol, 2009, 388 (1): 144-158; Peterson QP, et al. J Med Chem, 2009, 52 (18): 5721-5731.) Meanwhile, procaspasse-3 also functions as inducing apoptosis in a variety of tumor cells and is positively correlated with the expression of caspase-3 (Roy S, et al. Proc Natl Acad Sci USA, 2001, 98 (11): 6132-6137). Recent studies have also shown that high concentration of PAC-1 can induce apoptosis from the endoplasmic reticulum apoptotic pathway and show unique apoptotic characteristics (West DC, et al. Mol Pharm. 2012, 9 (5): 1425-1434).

Formula (I)

Chinese patent publication CN101184491A and US patent publication US 2007/0049602A1 disclosed the first small molecule compound PAC-1 which directly activated procaspasse-3 and claimed PAC-1, PAC-1 derivative library compounds including the related ones having the ZZ formula, ZZ2 formula, ZZ3 formula, ZZ4 formula and synthesis methods thereof; as well as claimed the method of selectively inducing apoptosis in cancer cells, the method of directly screening compounds to modify procaspasse-3 molecule in vitro and intracellular, the method of identifying or determining the potential sensitivity of cancer cells in response to the treatment of Caspase zymogen activating compounds, the method of screening for such candidate that may be suitable for the treatment of a caspase activator by identifying elevated levels of caspases in the cancer candidates. International patent WO 2010/091382 A1 claimed PAC-1, the non-neurotoxic compound S-PAC-1 and the analogs thereof.

PAC-1s currently reported in the literature are all in crystal form. Furthermore, it has been reported in the literature that the crystal form of PAC-1 is highly neurotoxic (see Q P. Peterson, D. C. Hsu, C J. Novotny, et al. Discovery and Canine Preclinical Assessment of a Nontoxic Procaspase-3-Activating Compound, Cancer Res 2010; 70:

7232-7241), which made the prospect of developing PAC-1 as a drug not favored.

SUMMARY OF THE INVENTION

The present inventors conducted intensive studies to solve the technical problem of high neurotoxicity by PAC-1 and its limited application as a medicament. As a result, PAC-1 in an amorphous form was prepared, and it is found that compared with the known crystal PAC-1, the neurotoxicity of PAC-1 in an amorphous form is significantly reduced so as to complete the present invention.

The present invention is as follows.

1. A compound PAC-1 represented by the following formula (I), which is in an amorphous form:

(I).

2. An oral pharmaceutical composition comprising the compound PAC-1 of claim 1 as the active ingredient, and a pharmaceutically acceptable carrier.
3. The oral pharmaceutical composition according to claim 2, which is a tablet, capsule, dripping pill or pill.
4. The oral pharmaceutical composition according to claim 2 or 3, further comprising a crystal form of PAC-1 or a salt thereof, wherein the amorphous form of PAC-1 is present in an amount of 10% or more by weight, preferably 50% or more, more preferably 80% or more, more preferably 90% or more, more preferably 95% or more, more preferably 97% or more, more preferably 99% or more, with respect to the total amount of the PAC-1 or a salt thereof in crystal form and the PAC-1 in amorphous form.
5. The oral pharmaceutical composition according to any one of claims 2 to 4, wherein the oral pharmaceutical composition is used for the treatment of a tumor, preferably a metastatic tumor.
6. The oral pharmaceutical composition according to any one of claims 2 to 4, wherein the oral pharmaceutical composition is used to inhibit tumor metastasis.
7. Use of the PAC-1 in amorphous form according to claim 1 in the preparation of an oral pharmaceutical composition.
8. The use according to claim 7, wherein the oral pharmaceutical composition is used for the treatment of a tumor, preferably a metastatic tumor.
9. The use according to claim 7, wherein the oral pharmaceutical composition is used to inhibit tumor metastasis.

According to the invention, PAC-1 in amorphous form shows significantly lower neurotoxicity than PAC-1 crystals and is therefore more suitable for the development of medicaments for the treatment of diseases such as tumors, in particular metastatic tumors.

BRIEF DESCRIPTION OF THE DRAWINGS

Figure 1. Chemical structure of PAC-1 and the analogs thereof.
Figure 2. Comparison of microscope images of PAC-1 needle crystal versus ZYS-1.
Figure 3. Comparison of scanning electron images of PAC-1 needle crystal versus ZYS-1.
Figure 4A.X-ray diffraction pattern of PAC-1 needle crystal
Figure 4B.X-ray diffraction pattern of ZYS-1 powder
Figure 5. Effect of ZYS-1 and PAC-1 needle crystal on HGC-27 cell proliferation
Figure 6. Effect of ZYS-1 and PAC-1 needle crystal on K562/A02 cell proliferation
Figure 7. Effect of ZYS-1 and PAC-1 needle crystal on A549 cell proliferation
Figure 8. Effect of ZYS-1 and PAC-1 needle crystal on HT-1080 cell proliferation
Figure 9. Effect of different doses of ZYS-1 on two-dimensional migration of HGC-27 cells (cell scratch assay)
Figure 10. Effects of different doses of ZYS-1 on three-dimensional migration of HGC-27 and HT-1080 cells (Transwell assay)
Figure 11. Effect of different doses of ZYS-1 on HT-1080 cell invasion (Transwell assay)

Figure 12. Effect of ZYS-1 on HUVEC cell proliferation and tubule formation. A: Effect of ZYS-1 on HUVEC cell proliferation; B, C: Effect of ZYS-1 on tubular formation.
Figure 13. Effect of different doses of ZYS-1 on Caspasc-3, NF-κB, VEGF and IXAP protein expression.

DETAILED DESCRIPTION OF THE INVENTION

Unless otherwise conflicting with the definitions in this specification, the terms in this specification have the meanings commonly understood by those skilled in the art, but in the cases of any conflict, the definitions in this specification shall prevail.

PAC-1

In the present specification, PAC-1 refers to a small molecule compound capable of directly activating Procaspase-3, which is named as 1-Piperazineacetic acid, 4-(phenylmethyl)-2-[[2-hydroxy- 3-(2-propen- 1-yl) phenyl] methylene] ((E)-N'-(3-allyl-2-hydroxybenzylidene)-2-(4-benzylpiperazine-yl)acethydrazide) and has the structure shown in Formula (I). It is believed that PAC-1 can selectively induce apoptosis in tumor cells and thus can be used as an anti-tumor drug.

(I)

PAC-1 in amorphous form

PAC-1s reported in the prior art all exist in crystal form and have strong neurotoxicity, which largely limits the application of PAC-1s as a medicament. The inventors unexpectedly discovered that PAC-1 in amorphous form showed significantly lower neurotoxicity than PAC-1 crystals. It should be noted that, in general, the amorphous form of a drug has a higher dispersion relative to the crystal form drug, which is more advantageous for the absorption of drugs, and correspondingly the toxicity should also stronger. However, in the present invention, it was unexpectedly found that PAC-1 in amorphous form (ie, ZYS-1) showed less toxicity. Therefore, the low neurotoxicity of PAC-1 in amorphous form is the technical effect that cannot be predicted by a person skilled in the art according to the prior art.

In the specification, PAC-1 in amorphous form, also known as ZYS-1, has the typical feature of the amorphous substance, that is, without any X-ray diffraction peak. In contrast, in some cases, PAC-1 in this description is also used to refer to PAC-1 crystal.

The ZYS-1 can be prepared, for example, by the following method, but the production method thereof is not limited thereto. Said method comprising:

Step (1): dissolving the PAC-1 crystal in an organic solvent to obtain a first solution of PAC-1;
Step (2): adding water to the solution of PAC-1 obtained in step (1) under stirring to obtain a second solution of PAC-1;
Step (3): continuously stirring until white or milky white solid precipitates, filtering, and removing the solvent to obtain the precipitate;
Step (4): Drying the resulting precipitate to give PAC-1 in amorphous form.

In step (1), the amount of the organic solvent may be, for example, 1 to 20 mL, preferably 2 to 10 mL, and more preferably 4 to 6 mL, with respect to 1 g of PAC-1 crystal. Ultrasound treatment, stirring, shaking and other means can be used to promote the dissolution of PAC-1 crystal. The organic solvent may be, for example, but not limited to, one or more selected from absolute ethanol, 95% ethanol, acetonitrile, diethyl ether, ethyl acetate, acetone and DMSO.

The ratio of the amount of water in step (2) to the amount of organic solvent used in step (1) may be but not limited to, for example, 1: 1 to 1:50, preferably 3: 1 to 20: 1, more preferably 5: 1 to 15: 1. The stirring speed may be but not limited to, for example, 100 to 5000 r/min, preferably 200 to 1000 r/min, more preferably 400 to 600 r/min.

In step (3), suction filtration may be used, which speeds up the filtration, but is not limited thereto. The suction filtration can be achieved by using a vacuum diaphragm pump, a water pump, an oil pump, or the like.

In the step (4), the drying is carried out under normal pressure or reduced pressure.

The resulting ZYS-1 can be confirmed by X-ray diffraction analysis.

Oral pharmaceutical compositions comprising ZYS-1

The inventors found that ZYS-1 showed significantly lower neurotoxicity than PAC-1 crystal in the case of oral administration. Therefore, ZYS-1 of the present invention may be preferably developed as an oral pharmaceutical composition.

The present invention provides an oral pharmaceutical composition comprising ZYS-1 as an active ingredient, preferably as the only active ingredient, and a pharmaceutically acceptable carrier. The crystal form of PAC-1 or a salt thereof may or may not be contained in the oral pharmaceutical composition of the present invention, and the crystal form of PAC-1 or a salt thereof may serve as an active ingredient, for example. In the case of comprising the crystal form of PAC-1 or a salt thereof, the amorphous form of PAC-1 is preferably present in an amount of 10% or more by weight, more preferably 50% or more, more preferably 90% or more, more preferably 95% or more, more preferably 97% or more, more preferably 99% or more, with respect to the total amount of the PAC-1 or a salt thereof in crystal form and the PAC-1 in amorphous form. The crystal form of PAC-1 or a salt thereof may be added to the oral pharmaceutical composition of the present invention intentionally or as an impurity entrained in the amorphous form of PAC-1.

The oral pharmaceutical composition of the present invention can be used for the treatment of tumors and/or tumor metastases. In particular, the oral pharmaceutical composition of the present invention can be used for the treatment of metastatic tumors. In this use, ZYS-1 of the present invention is preferred as the sole active ingredient.

In this specification, "treatment of a tumor" means treatment of a tumor in situ. "Treatment of tumor metastasis" refers to the treatment of metastatic tumor as well as the metastasis process, for example, inhibition of tumor metastasis. "Metastatic tumor" means that the tumor cells invade the lymphatic vessels, the blood vessels or other pathways from the primary site and are taken to other tissues, organs so as to continuely grow to form tumors or foreign tumors of the same type as the tumors of primary site. Said process is called metastasis, and the homogeneous or heterogeneous tumor formed is called metastatic tumor or metastatic carcinoma. In this process, the metastasis in the early stage needs a large number of new blood vessels to supply nutrition etc. required for its rapid growth. The whole process and the newly formed tumors are collectively referred to as metastatic tumors.

Therefore, the present invention also provides use of ZYS-1 for the preparation of an oral pharmaceutical composition, preferably the oral pharmaceutical composition for the treatment of tumors and/or tumor metastases, in particular the oral pharmaceutical composition for the treatment of metastatic tumors. In this use, ZYS-1 of the present invention serves as the active ingredient, preferably as the sole active ingredient.

As the pharmaceutically acceptable carrier, it can be conventionally selected by those skilled in the art according to the needs of the dosage form and the like. For example, the active ingredient may be dissolved or suspended in a suitable liquid (eg., water) in the preparation of an oral liquid formulation (liquid pharmaceutical composition). In the preparation of the oral solid preparation (solid pharmaceutical composition), the excipient and if needed the binder, disintegrant, lubricant, coloring agent, flavoring agent and the like may be added to the active ingredient so as to prepare tablets, coated tablets, granules, fine granules, powders, capsules or pills and the like according to a conventional method. As the excipient, for example, lactose, corn starch, sugar, glucose, sorbitol, crystalline cellulose, silica and the like can be used. As the binder, Polyvinyl alcohol, ethylcellulose, methylcellulose, acacia, hydroxypropylcellulose, hydroxypropylmethylcellulose and the like can be used. As the lubricants, magnesium stearate, talc, silica and the like can be used. As the coloring agent, the ones permitted to be employed in pharmaceuticals can be used. As the flavoring agent, cocoa, menthol, aromatic acid, peppermint oil, borneol, cinnamon powder and the like can be used. Of course, the above tablets, granules may also be coated with a sugar coating, a gelatin coating, and other necessary outer coatings.

Therapeutic regimens

The present invention also provides a method of treating tumors, in particular metastatic tumors, and the method of treating tumor metastasis (eg., inhibiting tumor metastasis), comprising the step of administering to a tumor patient the ZYS-1 of the invention described above, or the oral pharmaceutical composition comprising ZYS-1 described above. The ZYS-1 is preferably administered orally. In the case of administrating ZYS-1, or the oral pharmaceutical composition comprising ZYS-1 as described above, the dosage of ZYS-1 varies depending on the degree of symptoms, age of the patient, gender, body weight, sensitivity difference, administration route, administration time and duration of toxicity, the characteristic and type of the formulation, and the type of the active ingredient without any special restriction. Typically, the amount for an adult (body weight 60 kg) is 0.1-2000 mg, preferably 0.2-1000 mg, more preferably 1-500 mg per day, and the above-mentioned dosage can usually be divided into 1 to 6 times a day.

## Examples

### Example 1: Preparation of ZYS-1

1 g of PAC-1 needle crystal (prepared according to the method of Quinn P. Peterson et al., J. Med. Chem. 2009, 52, 5721-5731) was dissolved in DMSO (5 mL) with sonication. Under stirring at 500 r/min, 50 mL water was added and the stirring was continued for 10 min until the milky white precipitate appeared, filtered under reduced pressure and dried in vacuo to give a white solid.

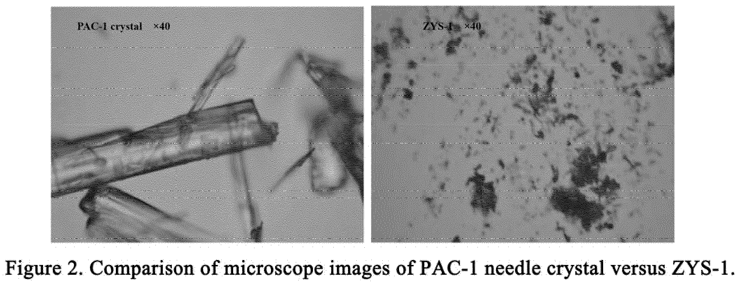

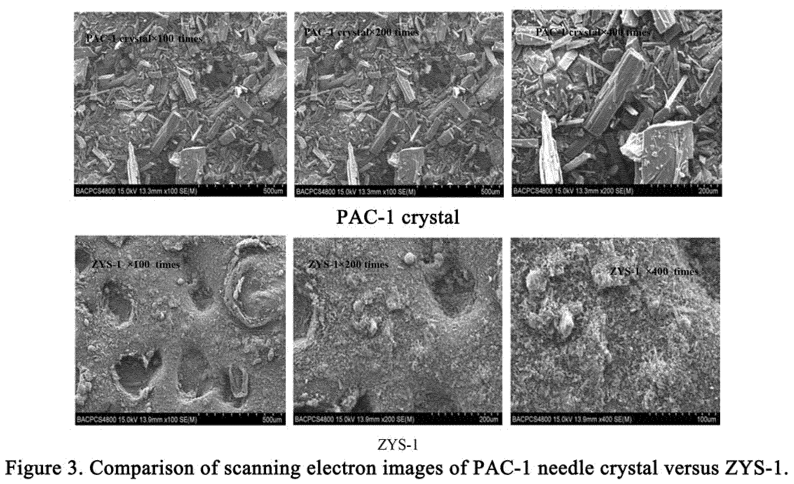

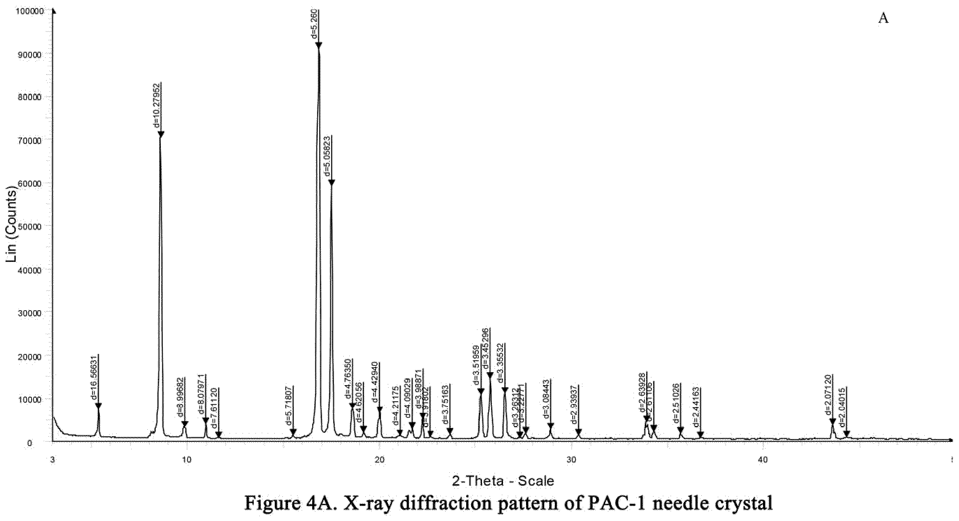

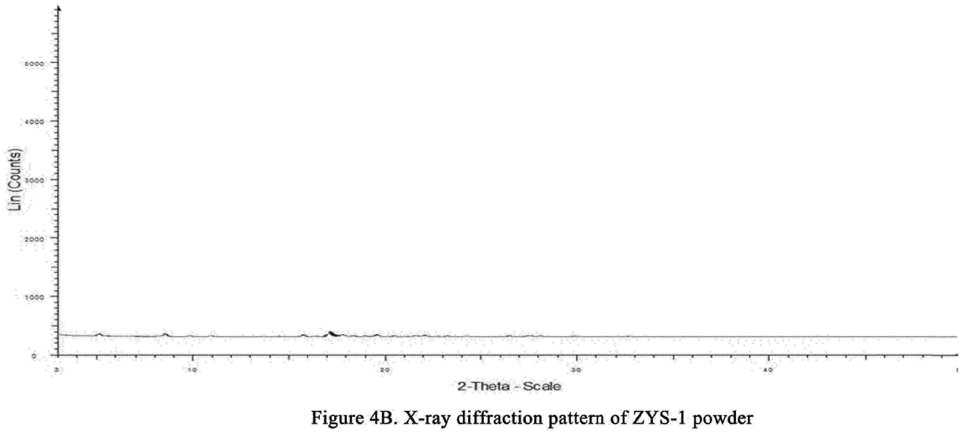

By microscopy, scanning electron microscopy and X-ray powder diffraction, it is clear that amorphous PAC-1 powder (ZYS-1) was obtained. Under the microscope and scanning electron microscope, it can be seen that the morphology of amorphous form and the needle crystal are completely different, and the needle crystal surface is relatively smooth with typical needle crystal, and ZYS-1 presented a loose, porous structure, as shown in Figure 2 -3. By comparing the X-ray diffraction patterns of the two, it can be seen that there is no obvious characteristic peak of ZYS-1, as shown in Fig.4A and Fig.4B.

### Example 2: Solubility Comparison ZYS-1 and PAC-1 Needle Crystal

The appropriate amounts of ZYS-1 and PAC-1 needle crysttal were dissolved into the appropriate amount of methanol, absolute ethanol, 95% ethanol, acetonitrile, ethyl ether, ethyl acetate, propanol, acetone, respectively, and sonicated for 1 hour, then left at room temperature for 24 hours to obtain the oversaturated solution. After centrifugation at 4000 r/min for 5 min, the supernatant was diluted appropriately and filtered through a 0.45 μm needle filter. The concentration was determined by HPLC and the solubility was calculated.

The experimental results showed that the solubility of ZYS-1 was higher than PAC-1 needle crystal, as shown in Table 1.

**Table 1. Solubility comparison of ZYS-1 and PAC-1 needle crystal**

| Solvent | PAC-1needle crystal (mg/mL) | ZYS-1 (mg/mL) |
|---|---|---|
| Methanol | 28.18 | 35.15 |
| Absolute ethanol | 18.95 | 23.53 |
| 95% ethanol | 16.45 | 18.51 |
| Propanol | 16.04 | 25.55 |
| Acetone | 81.77 | 128.97 |
| Chloroform | 290.29 | 344.73 |
| Diethyl ether | 3.51 | 6.31 |
| Acetonitrile | 20.74 | 28.74 |
| Ethyl acetate | 33.36 | 40.08 |
| Water | 0.0015 | 0.0056 |

### Example 3: In vivo pharmacokinetic comparison of ZYS-1 and PAC-1 needle crystal

Twelve male Wistar rats were randomly divided into ZYS-1 group and PAC-1 group. These rats were fasted for 12 hours before administration, and fed water freely. After ZYS-1 and PAC-1 needle crystal were given at the dose of 60mg/Kg, 0.5 mL orbital blood was collected at 0,5,20,30,45,60,120,180,240,360,480,720 mins, centrifuged at 5000 r/min for 5 min. 200 μL plasma was taken. The plasma was added 3 mL acetone and vortexed for 1 min. After centrifuging at 15000 r/min for 5 min, the supernatant was collected and dried under nitrogen at 37 ° C, to which 200 μL acetonitrile was added to be reconstituted. After sonication for 10 min, the mixture was vortexed for 1 min, and filted through 0.45 microporous filter membrane. The filtrate was tested by HPLC, and the plasma concentration at each time point was calculated. The curve of the plasma concentration curve over time was plotted, as shown in Table 2.

The results showed that ZYS-1 had higher in vivo bioavailability than PAC-1.

**Table 2. Pharmacokinetic parameters in rats (n=6)**

| Parameters | ZYS-1 | PAC-1 needle crystal |
|---|---|---|
| $k_a$(1/h) | 0.0324±0.0098 | 0.0720±0.0468 |
| $k_e$(1/h) | 0.0048±0.0033 | 0.0062±0.0029 |

(continued)

| Parameters | ZYS-1 | PAC-1 needle crystal |
|---|---|---|
| V/F(L) | 0.0308±0.0167 | 0.0318±0.0177 |
| T1/2ka(h) | 22.9129±6.3243 | 15.4260±11.8916 |
| T1/2a(h) | 209.5239±129.5666 | 134.7563±63.3796 |
| lagtime(h) | 4.6861±5.2184 | 8.8081±4.8496 |
| $T_{max}$(h) | 65.0000±9.4439 | 52.5000±12.5499 |
| $C_{max}$(μg/L) | 2194.2145±126.78 | 2078.7579±950.561 |
| AUC(0-tn)(μg/L*h) | 389290.2464±135976.41 | 320218.6357±127076.7162 |
| AUC(0-∞)(μg/L*h) | 724124.1563±206913.2939 | 578899.3071±466830.1720 |
| MRT(0-tn)(h) | 166.5375±51.4626 | 254.3166±180.2413 |
| MRT(0-∞)(h) | 420.4914±271.8423 | 1501.7189±1808.4213 |

Example 4: Preparation of ZYS-1 Ordinary Tablet

ZYS-1 20g, lactose 60g, starch 58g, dextrin 58g, silica powder 2g, magnesium stearate 2g were taken and mixed well. They were compressed into 1000 ZYS-1 ordinary tablets.

Example 5: Preparation of ZYS-1 Sustained Release Microspheres

First, 500 mL of a mixed aqueous solution containing 1.5% of sodium alginate (W/V), 1.5% of starch (W/V) and 10 mg of ZYS-1 was prepared. The solution was added 1000 mL of liquid paraffin as the oil phase, 30 mL mixture of Span-80 and Tween -80 (7: 1) as the emulsifier, and was stirred to form emulsion A.

Similarly, 100 mL 8% of $CaCl_2$ solution was prepared. The solution was added 300 mL liquid paraffin, 10 mLemulsifier (Span-80: Tween-80-7: 1) and appropriate amount of ethanol and was stirred to form emulsion B.

After both of the two emulsions became stable, the emulsion B was added to the emulsion A to perform crosslinking reaction for 15 min. The solution was centrifuged to obtain the ZYS-1 microspheres, which was sequentially washed with ethyl acetate, absolute ethanol and deionized water, and was allowed to dry at room temperature to obtain ZYS-1 sustained-release microspheres. It can be used to prepare tablets or capsules and other pharmaceutically acceptable forms of formulations.

Example 6: Acute Oral Toxicity Test in Mice with PAC-1

Experimental materials: PAC-1 needle crystal (prepared according to the method of Quinn P. Peterson et al., J. Med. Chem. 2009, 52, 5721-5731), batch number 20090818.

Experimental animals and feeding conditions: ICR mice, half male and half female. Body weight 20 ± 2g, provided by Beijing Weitonglihua Experimental Animal Technology Co., Ltd., SPF grade feeding, certificate: SCXK (Beijing) 2006-0009. Particulate feed for mouse breeding was provided by Beijing Ke'ao Xieli Feed Co., Ltd. Animals were housed in barrier-level (Grade 2) animal lab in Beijing Anzhen Hospital, fluorescent lighting, 12h light and dark cycle, room temperature 22 ~ 24 °C, humidity 40-47%, air supply 10-12 times / h, Laboratory license number: SYXK (Beijing) 2005-0028. Mice were housed in clear plastic boxes with 5 per cage separated by gender.

The method of preparing the test drug: PAC-1 needle crystal was placed in a mortar to be finely ground. A propriatc amount of PAC-1 crystal was accurately weighed, which was formulated with 0.5% CMC-Na to reach the desired concentration.

Animals grouping: After fasting (except for water) for 11 h, according to body weight,animals were randomly divided into groups of 10 with5 males and 5 females. There were 8 groups in total. I~VII are PAC-1 groups at different doses in which the interval among the dose groups of drug administration was 0., and the VIII group is 0.5% CMC-Na negative control group. Administration doses were 1000, 700, 490, 343, 240.1, 168.1, 117.1 mg/kg, respectively. Each administration group was dosed according to the body weight of the animals, and the administration volume was 0.4 mL/10 g body weight. One-time intragastric administration was used in the experiment. The negative control group was orally administered an equal volume of 0.5% CMC-Na. The animals started feeding at 3 hours after administration.

Observed indicators: immediately after administration, the observation of theresponses of the animals to drugs (such as: general performance, respiration, activity, with or without convulsions, righting reflex, pain reflex, response to external stimuli and other indicators) was started. When observing and recording the symptom of toxic reaction after animal administration, the time and duration of toxicity, recovery time, and the time of animal death, the animal was

immediately dissected grossly, visually observing the main organs (heart, liver, spleen, lung and kidney , stomach) to see if there is congestion, increased volume, hardens, discoloration. For survival animals, they were observed and recorded day by day whether there was delayed death and delayed toxicity reaction in 7 days. The animals were weighed and fed once every 2 days. After finishing observation for 7 days, the survival animals were dissected grossly and the situations of their main organs were observed. The number of vital animals in various dosage groups was calculated according to Bliss method to yield $LD_{50}$ (95% confidence limit).

Experimental results: The acute toxicity ($LD_{50}$) dose for PAC-1 one-time intragastric administration to mice was 551.61mg/kg, with 95% confidence limit C. L = 674.9703 ~ 450.8035mg/kg (results as shown in Table 3). Animals presented toxicity reactions 10min after oral intragastric administration of 1000mg/kg, and there were obvious neurotoxic symptoms, mainly manifested as significantly reduced spontaneous activity, prone, abdomen affixed with paroxysmal jumps, ataxia, tail upturned, drooling, and then clonic convulsions until opisthotonus, respiratory failure and death. The death occurred mainly within 30 ~ 60min after administration; the main toxicity reactions of the survival animals were the same as the animals at high dose, however, the toxicity was relatively low, which was considered to be neurotoxic symptoms. Most of the survival animals returned to normal within 3 h after dosing. The 100% mortality dose was greater than 1000 mg/kg; the 0% mortality dose was 240.1 mg/kg; and the dose without apparent toxicity was 117.7 mg/kg. The dead animals were dissected, and no obvious abnormalities were visually observed on major organs (heart, liver, spleen, lung, kidney, stomach, etc.). Other than the death occurred on the administration day, no death was record at other time. The survival animals were observed 7 days after administration, and no significant differences regarding the body weight gain, food intake, appearance, behavior, secretions, etc. were observed as compared to control groups. After the observation, the survival mice were grossly dissected, and the visual observation showed that a portion of animals' kidneys presented different lighter color degrees at 1000, 700, 490 and 343 mg / kg dosage groups. It was speculated that the drug may cause renal toxicity in a dose dependent manner, but kidneys coefficients at every group showed no statistical difference between the treatment groups and the control group. The remaining main organs showed no obvious abnormalities.

**Table 3. Effect of PAC-1 one-time intragastric administration to mice on acute toxicity (LD50)**

| Drug name | Grade | Dosage ( mg/kg) | Death number/total | Mortality rate % |
|---|---|---|---|---|
| PAC-1 | I | 1000 | 9/10 | 90 |
| | II | 700 | 6/10 | 60 |
| | III | 490 | 5/10 | 50 |
| | IV | 343 | 2/10 | 20 |
| | V | 240.1 | 0/10 | 0 |
| | VI | 168.1 | 0/10 | 0 |
| | VII | 117.7 | 0/10 | 0 |
| Negative Control group | VIII | - | 0/10 | 0 |

Example 7: Acute Toxicity Test with ZYS-1 Oral Administration in Mice

Experimental material: ZYS-1

Experimental animals and feeding conditions: ICR mice, half male and half female. Body weight 20 ± 2g, provided by Beijing Weitonglihua Experimental Animal Technology Co., Ltd., SPF grade feeding, certificate: SCXK (Beijing) 2006-0009. Particulate feed for mouse breeding was provided by Beijing Ke'ao Xieli Feed Co., Ltd. Animals were housed in barrier-level (Grade 2) animal lab in Beijing Anzhen Hospital, fluorescent lighting, 12h light and dark cycle, room temperature 22 ~ 24 °C, humidity 40-47%, air supply 10-12 times / h, Laboratory license number: SYXK (Beijing) 2005-0028. Mice were housed in clear plastic boxes with 5 per cage separated by gender.

Preparation method of test drug:A white powderof ZYS-1 was placed in a mortar to be finely ground. The drug was accurately weighed before use, which was formulated with 0.5% CMC-Na to reach the desired concentration.

Animals grouping: After fasting (except for water) for 11 h, according to body weight, animals were randomly divided into groups of 10 with 5 males and 5 females. There were 8 groups in total. I~VIII are ZYS-1 groups at different doses in which the interval among the dose groups of drug administration was 0.7, and the IX group is 0.5% CMC-Na negative control group. Administration doses were 4164.9, 2915.4, 2040.8, 1428.6, 1000, 700, 490, 343 mg/kg, respectively. Each administration group was dosed according to the body weight of the animals, and the administration volume was 0.4 mL/10 g body weight. One-time intragastric administration was used in the experiment. The negative control group was orally administered an equal volume of 0.5% CMC-Na. The animals started feeding at 3 hours after administration.

Observed indicators: immediately after administration, the observation of the responses of the animals to drugs (such as: general performance, respiration, activity, with or without convulsions, righting reflex, pain reflex, response to external stimuli and other indicators) was started. After observing and recording the symptom of toxic reaction, the time and duration of toxicity, recovery time, and the time of animal death. After the animal was dead, it was immediately grossly dissected, visually observing each of the main organs (heart, liver, spleen, lung and kidney , stomach) to see if there was congestion, increased volume, hardens, discoloration. The survival animals were observed and recorded day by day whether there was delayed toxicity reaction and delayed death within 7 days, and weigh and feed the animals once every 2 days. After finishing the 7 day's observation, the survival animals were grossly dissected and the situations of the main organs were observed. LD50 was calculated according to the number of animal deaths in each group.

Experimental results: On that administration day, in the 4164.9 mg/kg dosage group, animals showed toxicity reaction 15 mins after intragastric administration, and there were obvious neurotoxic symptoms, mainly manifested as significantly reduced spontaneous activity, prone, abdomen affixed with paroxysmal jumps, ataxia, tail upturned, drooling, and then clonic convulsions until opisthotonus, respiratory failure and death. The toxic reaction was obvious neurotoxic symptoms. The death occurred mainly within 6 hours after administration. The dose with no obvious toxicity reaction is 343mg/kg. The main toxicity reactions of animals in other dosage group are the same as the high dose group, however, with relatively lower toxicity, mainly manifested as neurotoxic symptoms. Most of the survival animals returned to normal within 12 h after administration. The dead animals were dissected, and no obvious abnormalities were visually observed on each of the major organs (heart, liver, spleen, lung, kidney, stomach, etc.) by naked eyes. The survival animals were observed 7 days after administration, and no significant differences regarding the body weight gain, food intake, appearance, behavior, secretions, etc. were observed as compared to controls. After the observation, the survival mice were grossly dissected, no significant difference can be seen on the main organs. The results were as shown in Table 4.

**Table 4. Effect of ZYS-1 one-time intragastric administration on acute toxicity (LD50) in mice**

| Drug name | Group | Dosage ( mg/kg) | Death number/total | Mortality rate % |
|---|---|---|---|---|
| ZYS-1 | 1 | 4164.9 | 10/10 | 100 |
| | 2 | 2915.4 | 7/10 | 70 |
| | 3 | 2040.8 | 5/10 | 50 |
| | 4 | 1428.6 | 5/10 | 50 |
| | 5 | 1000 | 4/10 | 40 |
| | 6 | 700 | 3/10 | 30 |
| | 7 | 490 | 2/10 | 20 |
| | 8 | 343 | 0/10 | 0 |
| Negative Control | 9 | - | 0/10 | 0 |

The LD50 dose of ZYS-1 byone-time intragastrical administration was 1665.7 mg/kg, with 95% confidence limit C.L = 1091.8 ~ 2285.3 mg/kg; the 0% mortality doseis 343 mg/kg; non-significant toxic reaction dose is 343 mg/kg.

Comparing the experimental results of acute toxicity of ZYS-1 and PAC-1, it can be seen that ZYS-1 has lower neurotoxicity.

Example 8: Comparison of Antitumor Pharmacodynamics of ZYS-1 and PAC-1 Needle Crystal

Experimental cells: gastric cancer cell line HGC-27, non-small cell lung cancer A549, human chronic myeloid leukemia cells doxorubicin resistant strains K562/A02, human fibrosarcoma HT-1080, human chronic myeloid leukemia cell K562.

Experimental materials: PAC-1 needle crystal (prepared according to the method described in Quinn P. Peterson et al., J. Med. Chem. 2009, 52, 5721-5731), MTT solution (Amresco specification: 1 g, batch number: 2035B358), DMSO (Analytical Pure, Beijing Chemical Factory), 96-well plates (Corning), CCK-8 (Japanese Tongren Chemical Research Institute, specification: 500test, batch number: DJ706), fetal bovine serum was the product from the United States GIBCO, batch number: 608759, RPMI-1640 is the product of Hyclone, batch number: NWK0492, 0.25% Trypsin-EDTA is the product from the United States GIBCO company, batch number: 10T774768, DMSO: USA Sigma Corporation.

The cells in logarithmic growth phase were collected. The cells was digested and the cell density was adjusted into 1-5 x $10^5$ cells/mL, 100 $\mu$L per well in 96-well culture plate. The plated was cultured in a incubator with 37 °C, 5% CO2 saturated humidity. After adherent, different concentrations of PAC-1 and ZYS-1 were added for dosing groups. 6 dosage groups were set, with at least three parallel wells set in each group. Also, the negative control group (containing

0.05% DMSO medium), positive control group (20 μM etoposide), and blank control group (without cells, medium only). They were cultured in the incubator with 37 °C, 5% CO2 saturated humidity. After 48 h, 20 μL MTT (5 mg/mL) or 10 μL CCK-8 solution was separately added to each well, incubated at 37°C and shaked for 10 min to completely dissolve formazan. the optical density (OD) value was determined by a microplate reader under the condition of the corresponding detection wavelength, and the inhibition rate and IC50 of the drug were calculated by the following formula:

$$\text{Inhibition rate} = (\text{OD value of the negative control group - OD value of the dosing group})/(\text{ OD value of the negative control group - OD value of the blank group}) \times 100\%$$

The experiment was replicated 3 times.

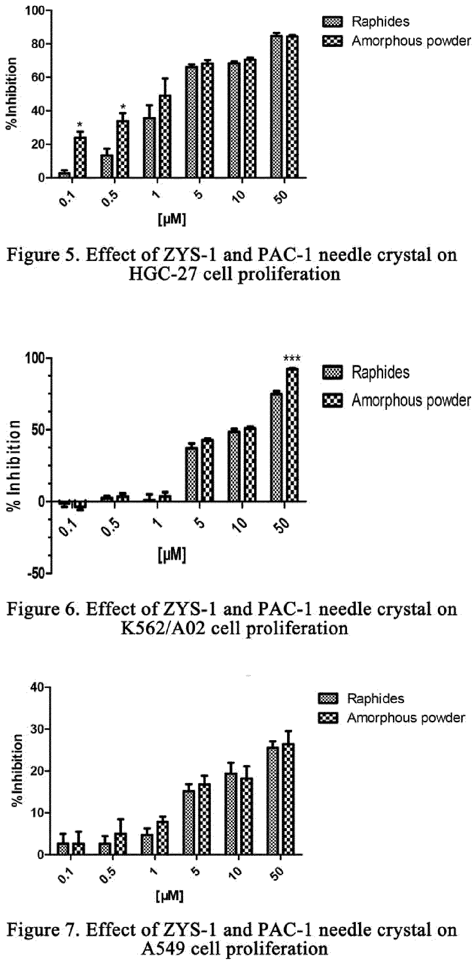

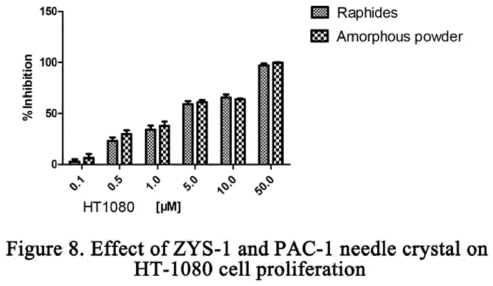

The results were shown that ZYS-1 has stronger antitumor efficacy than PAC-1 needle crystal, as presented in Figure 5-8.

Example 9: ZYS-1 inhibiting two-dimensional migration of HGC-27 cells

Experimental materials: the same as Example 8.

HGC-27 cells in logarithmic growth phase were collected. The cell with density of $5 \times 10^5$ cells/mL was spread onto 24-well plates (0.5 mL per well). RMPI-1640 culture medium with 10% fetal bovine serum was added and cultured for 24 h to form monolayer cells. The monolayer cells were scratched in a line by 20 μL pipette gun tip, which was washed with PBS three times. Then, drug-containing media with 2% fetal bovine serum s were added, which concentrations were 2μM, 4 μM, respectively. The media were cultured in the incubator with 37 °C, 5% CO2 saturated humidity. Sampled at 0, 4, 8, 12 h and took the pictures. The area where cells were migrated from scratch edge to scratch within the same field was counted by software.

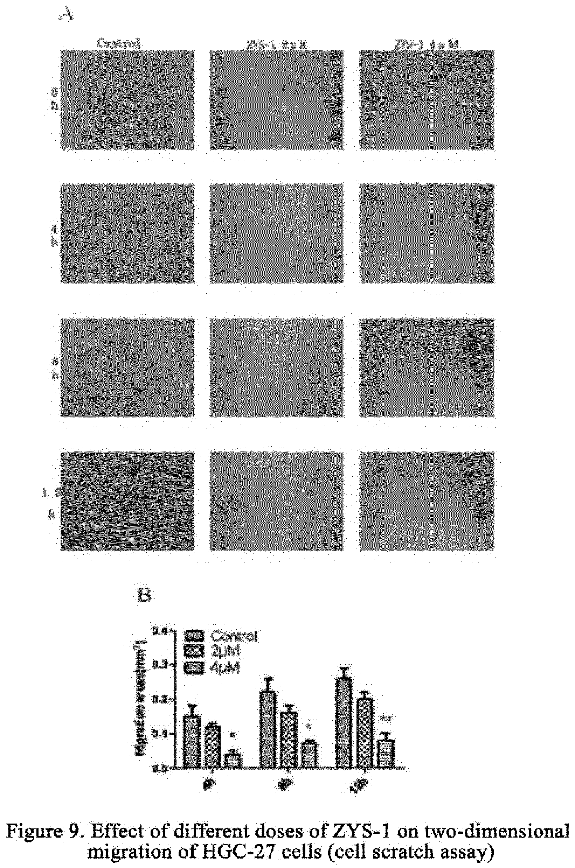

The test results were shown in Figure 9 and Table 5. The scratch test results showed that ZYS-1 had a significant inhibitory effect on HGC-27 cell migration.

**Table 5. Effect of ZYS-1 on the two-dimensional migration of HGC-27 cells n=3 (X ±SD)**

| Groups | 4h (mm$^2$) | 8h (mm$^2$) | 12h (mm$^2$) |
|---|---|---|---|
| Control | 0.15±0.03 | 0.22±0.04 | 0.26±0.03 |
| 2μM | 0.12±0.01 | 0.16±0.02 | 0.20±0.02 |
| 4μM | 0.04±0.01* | 0.07±0.01* | 0.08±0.02** |
| Compared with the control group * P <0.05, ** P <0.01 | | | |

Example 10: ZYS-1 inhibiting three-dimensional migration of HGC-27 and HT-1080 cells

Experimental materials: Transwell (Corning Incorporated costar, item No.: 3422, batch number: 27911039 specifications); the rest *ibid.*

Starved cells were digested and collected, which were suspended in serum-free medium. The cell intensity was adjusted into $3 \times 10^5$ cells/mL. 100 μL of the cell suspension was added into the upper chamber and 700 μL of medium with 10% serum to the lower chamber per well. According to the experimental design, 50 μL serum-free medium was added per well for the control group, 50 μL of drug-containing serum-free medium was added for the administration groups. They were continued to be cultured in the incubator for another 24 h. The upper chamber was taken, with methanol fixation for 30 min, dyed with Giemsa stain for 30 min, rinsed with clean water twice. The cells on the bottom membrane surface of the upper chamber were carefully wiped off with a wet cotton swab, dried in the air. With the bottom up, they were transferred onto glass slide and sealed with neutral gum. Under the microscope, nine random fields were taken and the transmembrane cells therein were counted. The average values were statistically calculated.

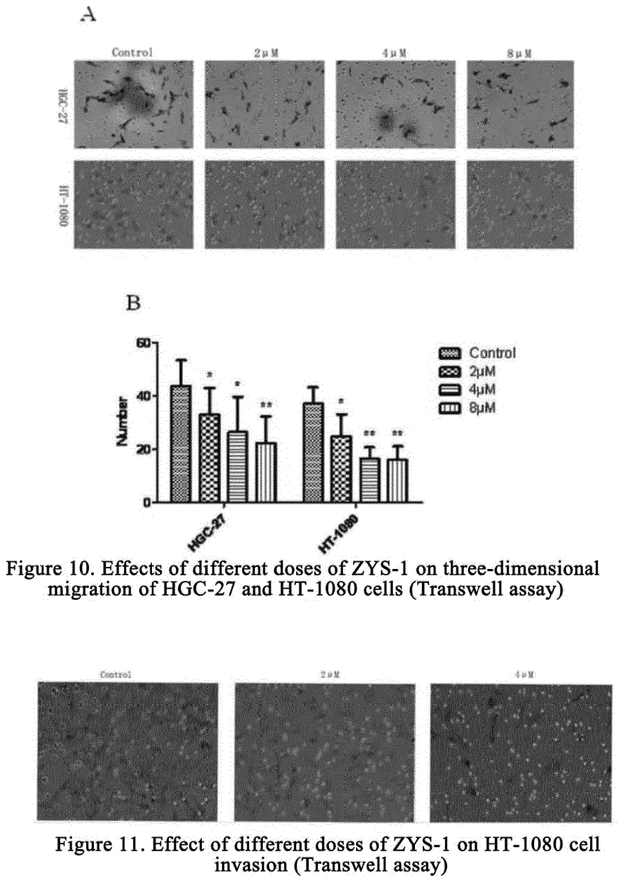

The results showed that ZYS-1 could significantly inhibit the three-dimensional migration ability of HGC-27 and HT-1080 cells in a dose-dependent manner, and ZYS-1 has a strong inhibitory effect on HT-1080, as presented in Figure 10 and Tables 6-7.

**Table 6. Effect of ZYS-1 on the Three-dimensional Migration Ability of HGC-27 Cells n=9 (X ±SD)**

| Groups | Conc. (μM) | Cell number | Inhibition Rate (%) |
|---|---|---|---|
| Control | - | 43.75±9.74 | 0.00 |
| | 2 | 33.00±10.00* | 24.57 |
| ZYS-1 | 4 | 26.30±13.37* | 39.89 |
| | 8 | 22.44±9.82** | 48.71 |
| Compared with the control group * P <0.05, ** P <0.01 | | | |

**Table 7. Effects of ZYS-1 on the Three-dimensional Migration Ability of HT-1080 Cells n=9 (X ±SD)**

| Groups | Conc. (μM) | Cell number | Inhibition Rate (%) |
|---|---|---|---|
| Control | - | 37.08±6.11 | 0.00 |
| | 2 | 24.67±8.46* | 33.47 |
| ZYS-1 | 4 | 16.58±4.18** | 55.29 |
| | 8 | 16.08±5.07** | 56.64 |
| Compared with the control group * P <0.05, ** P <0.01 | | | |

Example 11: ZYS-1 inhibiting HT-1080 cell invasion

Experimental materials: Transwell (Corning Incorporated costar, item No: 3422, batch number: 27911039, specification: 8.0 μm, 24-well plate); Matrigel™ matrigel (BD company, batch number: 31425; specification: 5ml, Item No: 356234); the rest *ibid.*

The matrigel was thawed at 4 ° C overnight and diluted with serum-free medium precooled at 4 °C at 1: 2. 30 μL of the diluted matrigel was added vertically in the center of the bottom of the upper chamber, allowed to stand at r.m. for 20 min, and then incubated at 37 ° C for 60 min. It was observed under the microscope until gelling, hydrated with the serum-free medium for use. The treated tumor cells with drugs for 48 h were digested and collected, and then the cells were suspended with serum free medium. The cell intensity was adjusted into 2.5 × $10^5$ cells/mL, and kept cultured in the incubator for another 24 h. The upper chamber was taken, with methanol fixation for 30 min, dyed with Giemsa stain for 30 min, rinsed with clean water twice. The cells on the bottom membrane surface of the upper chamber were carefully wiped off with a wet cotton swab, dried in the air. With the bottom up, they were transferred onto glass slide and sealed with neutral gum. Under the microscope, nine random fields were taken and the transmembrane cells therein were counted. The average values were statistically calculated.

The results showed that ZYS-1 could significantly inhibit the invasion ability of HT-1080 cells in a dose-dependent manner. A large number of cells from the HT-1080 control group invaded into the lower chamber, and the numbers of invasive cells from the 2 μM and 4 μM administration groups were smaller than those from the control group. No cell from 8μM administration group invaded into the lower chamber under the same conditions, as shown in Figure 11.

Example 12: Effect of ZYS-1 on HUVEC viability and tubule formation

Experimental materials: ECM medium (Sciencell Corporation, batch number: 9391, specification: 500 mL, Item number: 1001), Matrigel ™ matrigel (BD company, batch number: 31425; specification: 5mL, item number: 356234), 0.25% Trypsin-EDTA (US GIBCO company's product, specification: 5 mL, batch number: 10T774768), recombinant human endostatin injection (Shandong XianSheng Maidejin Pharmaceutical Co., Ltd., batch number: 201205015, specification: 3 mL), MTT (Amresco, specification: 1g, batch number: 2035B358), DMSO (analytical pure, Beijing Chemical Factory), 96 wells (Corning), triple distilled water, polylysine (PLL); the rest *ibid.*

Test Methods: The effect of ZYS-1 on the viability of HUVECs was determined by MTT method. The HUVECs in logarithmic growth phase were taken and digested with 0.25% trypsin, centrifuged and resuspended in ECM complete medium to formulate into 1 × $10^5$ cells/mL single cell suspension. The cells were seeded in 96-well culture plates at a density of 1 × $10^4$ cells/well, with a volume of 100 μL per well. They were placed and cultured in the incubator with 37 °C, 5% CO2. After culturing 24 h, six different concentrations of ZYS-1 solution were added to obtain the final concentrations of drugs at 0.1, 0.5, 1, 5, 10, 50 and 100 μM. Meanwhile, the positive control group was set as etoposide at a final concentration of 10 μM, and the negative control was an equal amount of 0.1% DMSO solution. Each concentration

of the experimental groups and the control groups all were set five replicates, with 100 μL of drugs per well. The blank control group is additionally set as 200 μL complete medium. The cells were placed and cultured in the incubator with 37 °C, 5% CO2. After culturing for 48h, 20 μL MTT solution (5 mg/mL) was added to each well, which was continued to be cultured for another 4 h. The culturing was terminated, and the culture supernatant in the well was carefully discarded. 150 μL DMSO was added into each well, shaked for 10 min, causing the crystal to be sufficiently dissolved. The light absorbance value of each well was measured by enzyme-linked immunosorbent assay. The measured wavelength was 570 nm and the result was recorded. The experiment was replicated three times.

Tubule formation assay: the matrigel was thawed at 4 °C overnight, which was 1: 1 diluted with serum-free ECM medium and coated onto 96-wcll plates. The diluted matrigel was sucked by precooled pipette gun tip, which was added into pre-cooled 96-well plates, with 50 μL per well. The matrigel was placed on ice for 5 min to make the matrigel surface level, and then the 96-well plates were put into a cell incubator at 37 °C for 1 h to cause gel coagulation. Trypsinize cells, the cells were counted and diluted with ECM medium. The cell density was adjusted to $3 \times 10^5$ cells/mL, and the cells were seeded into coated 96-well plates with 100 μL per well. According to the MTT results, the ZYS-1 solution at different concentrations were formulated. 100 μL of the solutions were taken and added into the cell-seeded wells to make the final concentration at 2, 1, 0.5 μM. At the same time, the positive control was set as recombinant human endostatin (Endostar) and the negative control as without drugs but medium only. Each concentration of the experimental groups and the control groups all were set three replicates,, which were placed and cultured in the incubator. After culturing 18 h, the culture was observed under microscope and taken the pictures to record the circumference, area of the generated tubes and the number of nodes as indicators for the formation of tubule. The experiment was replicated three times.

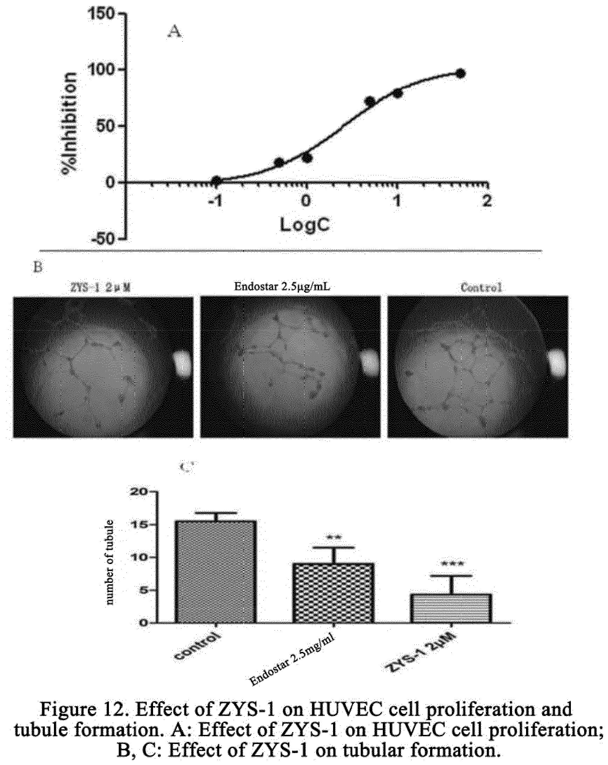

Conclusion: The results of MTT assay showed that ZYS-1 significantly inhibited HUVECs cells with IC50 of 2.542 μM in a dose-dependent manner, as shown in Figure 12A. The results of tubule formation assay showed that ZYS-1 had a strong inhibitory effect on the formation of endothelial cell tubules induced in vitro. The inhibition was similar between the 2 μM ZYS-1 group and the positive control 2.5 μg/mL Endostar, as shown in Figs. 12B and 12C.

Example 13: Effect of ZYS-1 on the Expression of NF-κB, XIAP, Caspase-3 and VEGF

Experimental materials: Rabbit anti-XIAP polyclonal antibody (batch number: 909098W) was purchased from Beijing Biosynthesis Biotechnology Co., Ltd.; rabbit anti-VEGF polyclonal antibody (batch number: 10CM199), rabbit anti-Caspase-3 polyclonal antibody, Rabbit anti-NF-κB polyclonal antibody (batch number: D-C1-07C14B), SABC-FITC kit (batch number: 06F10AN) were all purchased from Wuhan Boster Biotechnology Co., Ltd .; paraformaldehyde (Beijing Chemical Reagents Company, batch number: 060221); the rest *ibid.*

The sterilized cell transfer membrane was taken and placed into a 24-well plate, which was seeded with 1 mL ($1 \times 10^5$ cells/mL) cell suspension, and cultured for 24 h. Drug-containing medium was added, The negative control groups and administration groups were set, with the experimental groups intervened for 24-48 h. The plate was washed with PBS, and fixed with 4% paraformaldehyde for 15 min. It was treated with 0.1% TritonX-100, and washed with PBS, blocked with 1: 10 diluted normal serum blocking solution for 15 min. The plate was added 1: 100 diluted primary antibody, placed at 4 ° C overnight. The plate was washed with PBS, to which 1: 100 diluted secondary antibodies was added and incubated at room temperature for 30 min. The plate was washed with PBS, to which 1: 100 diluted SABC-FITC was added, and re-incubated at room temperature for additional 30 min. The unbound FITC was washed with PBS. We observed and taken pictures under fluorescence microscope, and analyzed the fluorescence intensity by Bioflux Montage software. 6 fields were selected randomly under microscope for each group and the integral optical density value of cells was calculated.

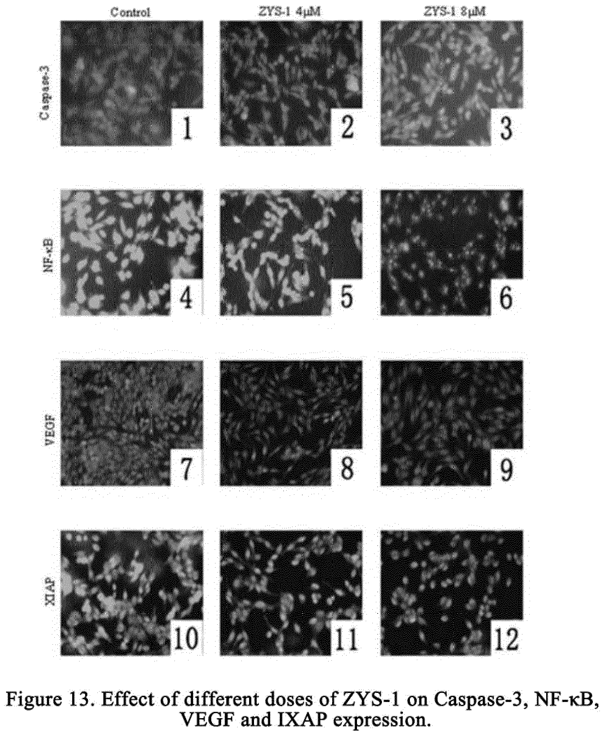

The experimental results showed that ZYS-1 could significantly inhibit the expression of NF-κB and XIAP, and increase the expression of caspase-3. Therefore, the apoptosis of HGC-27 cells induced by ZYS-1 may be achieved by down-regulation of NF-κB protein expression so as to reduce the expression of XIAP protein at transcription level and decrease its inhibitory effect on caspase family members, thereby increasing caspase pathway-dependent apoptosis. In addition, ZYS-1 significantly inhibited the expression of VEGF protein, suggesting that ZYS-1 could inhibit the occurrence and development of metastatic tumors. See Figure 13 and Table 8.

**Table 8. Effect of ZYS-1 on the protein expression of NF-κB, XIAP, caspase-3 and VEGF in HGC-27 cells n=6 (X ±SD)**

| | NF-κB | XIAP | Caspase-3 | VEGF |
|---|---|---|---|---|
| Control | 53.28±1.01 | 41.85±1.50 | 35.55±0.63 | 34.04±0.20 |
| 4μM | 43.83±1.06* | 38.22±0.81* | 37.26±0.39* | 31.17±0.24* |

(continued)

| | NF-κB | XIAP | Caspase-3 | VEGF |
|---|---|---|---|---|
| 8μM | 40.94±0.46* | 36.27±1.03* | 39.36±1.20* | 30.51±0.32* |
| Compared with the control group * P <0.05 | | | | |

Example 14: ZYS-1 inhibiting spontaneous metastatic cancer in mice

Experimental materials: Murine Lewis lung carcinoma cell line, C57BL/6 mice, SPF grade, half male and half female, 18-22g

Experimental methods: The concentration of cell suspension was adjusted to $5\times10^6$ cells/mL. The armpit of the right forelimb of the mice was inoculated with 0.2 mL per mice. The tumors growed to 1000 $mm^3$ and then were randomly divided into groups for adminstration, the groups were treated after three weeks. A total of 4 groups including ZYS-1 high, medium and low (100, 50, 25 mg/kg/d, intragastric administration) and positive control (gefitinib 100 mg/kg/d, intragastric administration) groups were set. The efficacy of ZYS-1 was evaluated in terms of general survival status (food intake, activity, hair etc.), tumor inhibition rate and metastasis inhibition rate.

Wherein the inhibition rate was calculated by the following method: the length and width of the tumor were measured with a vernier scale and the volume of the tumor was calculated (once a week), the tumor volume = length $\times$ width $^2$ $\times$ 0.52. The curve of tumor growth was plotted. On the next day after completing the treatment cycle, the animal was weighed. After collecting the blood,the animal was sacrificed. The tumor tissue was stripped, weighed, and the tumor inhibition rate was calculated. Store at -80?.

The tumor inhibition rate (%) = (1 - the average tumor weight of the experimental group/average tumor weight of the control group) $\times$ 100%, in which the inhibition of metastasis rate was calculated by the following method:: the lung was taken and weighed, the number of metastases loci on the positive and negative sides of the lung and that between lobes of the lung was counted.

Metastasis rate = Number of mice with metastasis/Total number of mice × 100%

Inhibition rate of metastasis loci = (average number of metastasis loci in the control group - average number of metastasis loci in the experimental group)/average number of metastasis loci in the control group × 100%

Experimental results:

1. General observation of animals

Tumor formation rate: about 3 days after inoculation of tumor cell solution, nodules in the armpit can be touched in some mice. One week after inoculation, all the inoculated mice showed massive tumors with a tumorigenic rate of 100%.

Food intake: The tumor-bearing mice had reduced food intake, wherein the low and medium dose showed comparative amount with the model group, mice with high dose were superior to the model group.

Activities: The activity of tumor-bearing mice decreased, wherein the mice in the model group and low dose group appeared fatigue and less active, mice in high and medium doses were superior.

Hair Condition: Mice administrated with high and medium dose of ZYS-1 had shiny coat, and vertical hair was not obvious, followed by low dose. Mice in the model group and the positive control group showed dull hair, and vertical hair was obvious.

The tumor status and tumor inhibition rate of the tumor bearing mice in each group were shown in Tables 9-10.

Table 9. Tumor weight and tumor inhibition rate of mice in each group

| Group | Dosage ( mg/kg) | Animal Number | | Body weight of the animals (g,X±SD) | | Tumor weight (g, X±SD) | tumor inhibit ion rate (%) | P value |
|---|---|---|---|---|---|---|---|---|
| | | Begi n | End | Begin | End | | | |
| Model | 0 | 24 | 18 | 21.5±1.7 | 35.0±4.0 | 3.9657±0.7639 | | |
| High | 100 | 12 | 12 | 21.9±1.8 | 34.1±4.2 | 1.0209±0.7332 | 74.26 | P<0.001 |
| Medium | 50 | 12 | 11 | 21.8±1.8 | 33.8±5.6 | 1.5844±0.8288 | 60.05 | P<0.001 |
| Low | 25 | 12 | 11 | 22.0±1.8 | 35.7±6.3 | 1.9579±0.8346 | 50.63 | P<0.01 |
| Gefitini b | 100 | 12 | 10 | 21.4±1.0 | 30.5±5.0 | 2.1036±0.3382 | 46.96 | P<0.001 |
| P<0.001, a very significant difference compared to the model group; P<0.01, a significant difference compared to the model group | | | | | | | | |

Table 10. Inhibition Effect of ZYS-1 on tumor metastasis in mice

| Group | Dosage (mg/kg) | Animal Number Begin | Number of animals End | Metastasis rate | Number of lung | | | P value |
|---|---|---|---|---|---|---|---|---|
| Model | 0 | 24 | 18 | 24 | 100% | 13.9±2.4 | | |
| High | 100 | 12 | 12 | 12 | 100% | 3.8±1.9 | 72.66 | P<0.001 |
| Mediu | 50 | 12 | 11 | 12 | 100% | 5.4±2.6 | 61.15 | P<0.001 |
| Low | 25 | 12 | 11 | 12 | 100% | 6.3±3.3 | 54.68 | P<0.01 |
| Gefiti nib | 100 | 12 | 10 | 12 | 100% | 6.9±3.7 | 50.36 | P<0.001 |
| P<0.001, a very significant difference compared to the model group; P<0.01, a significant difference compared to the model group | | | | | | | | |

The above results showed that ZYS-1 had good antitumor and anti-metastatic effects, for example, at the dose of 100 mg/kg, the inhibition rate of tumor metastasis was more than 70%, and with a good dose-dependent relationship.

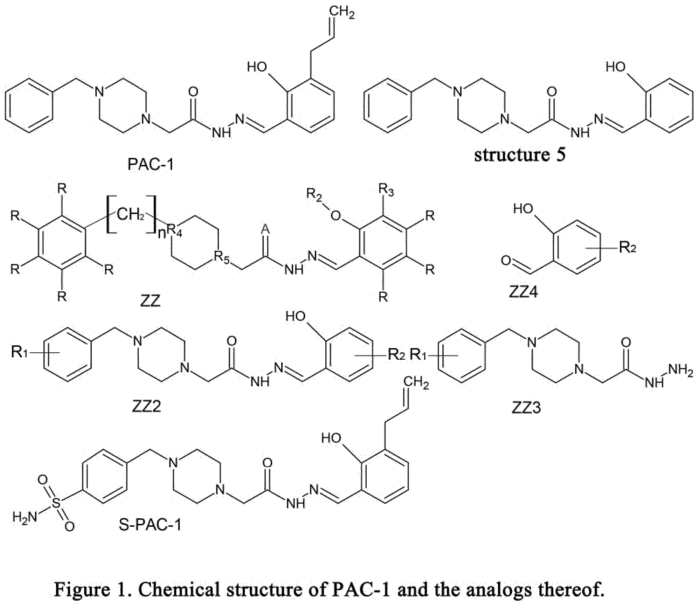

After reading the disclosure of this specification, one skilled in the art will readily recognize that the amorphous form of PAC-1 analogs or derivatives thereof (eg, salts, esters, solvates and the like) may also have low neurotoxicity. Under the teachings of the present specification, it is obvious for one of skill in the art to be fully motivated to obtain the analogs or derivatives of PAC-1 described above and to verify their biological toxicity, thereby obtaining an analog or derivative of PAC-1 that is less neurotoxic. Examples of the PAC-1 analogs include those disclosed in Chinese Patent Publication No. CN101184491A, such as PAC-1 derivative library compounds, compounds of ZZ formula, ZZ2 formula, ZZ3 formula, ZZ4 formula (see FIG. 1).

## Claims

**1.** A compound PAC-1 represented by the following formula (I), which is in an amorphous form:

(I).

**2.** An oral pharmaceutical composition comprising the compound PAC-1 of claim 1 as the active ingredient, and a pharmaceutically acceptable carrier.

3. The oral pharmaceutical composition according to claim 2, which is a tablet, capsule or pill.

4. The oral pharmaceutical composition according to claim 2, which is a dripping pill.

5. The oral pharmaceutical composition according to claim 2, further comprising a crystal form of PAC-1 or a salt thereof, wherein the amorphous form of PAC-1 is present in an amount of 10% or more by weight, preferably 50% or more, more preferably 80% or more, more preferably 90% or more, more preferably 95% or more, more preferably 97% or more, more preferably 99% or more, with respect to the total amount of the PAC-1 or a salt thereof in crystal form and the PAC-1 in amorphous form.

6. The oral pharmaceutical composition according to any one of claims 2 to 5 for use in the treatment of a tumor, preferably a metastatic tumor.

7. The oral pharmaceutical composition according to any one of claims 2 to 5 for use in the inhibition of tumor metastasis.

8. Use of the PAC-1 in amorphous form according to claim 1 in the preparation of an oral pharmaceutical composition.

9. The use according to claim 8, wherein the oral pharmaceutical composition is a composition for treatment of a tumor.

10. The use according to claim 8, wherein the oral pharmaceutical composition is a composition for treatment of a metastatic tumor.

11. The use according to claim 8, wherein the oral pharmaceutical composition is a composition for inhibition of tumor metastasis.

12. A compound PAC-1 represented by the following formula (I) for use in the treatment of a tumor, preferably a metastatic tumor or for use in the inhibition of tumor metastasis, wherein the compound is in an amorphous form:

HO O N N N N H (I).

**Patentansprüche**

1. Verbindung PAC-1, welche durch die folgende Formel (I) dargestellt ist, welche in einer amorphen Form vorliegt:

HO O N N N N H (I).

2. Orale pharmazeutische Zusammensetzung umfassend die Verbindung PAC-1 nach Anspruch 1 als den aktiven Inhaltsstoff, und einen pharmazeutisch verträglichen Träger.

3. Orale pharmazeutische Zusammensetzung nach Anspruch 2, welche eine Tablette, Kapsel oder Pille ist.

4. Orale pharmazeutische Zusammensetzung nach Anspruch 2, welche eine Drippingpille ist.

5. Orale pharmazeutische Zusammensetzung nach Anspruch 2 ferner umfassend eine Kristallform von PAC-1 oder ein Salz davon, wobei die amorphe Form von PAC-1 in einem Massenanteil von 10 Gewichtsprozent oder mehr,

bevorzugt 50 Gewichtsprozent oder mehr, bevorzugter 80 Gewichtsprozent oder mehr, bevorzugter 90 Gewichtsprozent oder mehr, bevorzugter 95 Gewichtsprozent oder mehr, bevorzugter 97 Gewichtsprozent oder mehr, bevorzugter 99 Gewichtsprozent oder mehr, bezogen auf die Gesamtmenge des PAC-1 oder eines Salzes davon in Kristallform und das PAC-1 in amorpher Form.

**6.** Orale pharmazeutische Zusammensetzung nach einem der Ansprüche 2 bis 5 zur Verwendung in der Behandlung eines Tumors, bevorzugt eines metastatischen Tumors.

**7.** Orale pharmazeutische Zusammensetzung nach einem der Ansprüche 2 bis 5 zur Verwendung in der Inhibierung einer Tumormetastase.

**8.** Verwendung des PAC-1 in amorpher Form gemäß Anspruch 1 in der Herstellung einer oralen pharmazeutischen Zusammensetzung.

**9.** Verwendung nach Anspruch 8, wobei die orale pharmazeutische Zusammensetzung eine Zusammensetzung zur Behandlung eines Tumors ist.

**10.** Verwendung nach Anspruch 8, wobei die orale pharmazeutische Zusammensetzung eine Zusammensetzung zur Behandlung eines metastatischen Tumors ist.

**11.** Verwendung nach Anspruch 8, wobei die orale pharmazeutische Zusammensetzung eine Zusammensetzung zur Inhibierung einer Tumormetastase ist.

**12.** Verbindung PAC-1, welche dargestellt ist durch die folgende Formel (I), zur Verwendung in der Behandlung eines Tumors, bevorzugt eines metastatischen Tumors, wobei die Verbindung in einer amorphen Form vorliegt:

HO O N N H N N (I).

## Revendications

**1.** Composé PAC-1 représenté par la formule (I) suivante, qui est sous forme amorphe :

HO O N N H N N (I).

**2.** Composition pharmaceutique orale comprenant le composé PAC-1 de la revendication 1 en tant que l'ingrédient actif, et un support pharmaceutiquement acceptable.

**3.** La composition pharmaceutique orale selon la revendication 2, laquelle est un comprimé, une capsule ou une pilule.

**4.** La composition pharmaceutique orale selon la revendication 2, laquelle est une pilule formée par égouttage.

**5.** La composition pharmaceutique orale selon la revendication 2, comprenant en outre une forme de cristal de PAC-1 ou un sel de celui-ci, sachant que la forme amorphe de PAC-1 est présente dans une quantité de 10 % ou plus en poids, de préférence 50 % ou plus, de façon plus préférentielle 80 % ou plus, de façon plus préférentielle 90 % ou plus, de façon plus préférentielle 95 % ou plus, de façon plus préférentielle 97 % ou plus, de façon plus préférentielle

99 % ou plus, par rapport à la quantité totale du PAC-1 ou d'un sel de celui-ci sous forme de cristal et du PAC-1 sous forme amorphe.

6. La composition pharmaceutique orale selon l'une quelconque des revendications 2 à 5 pour utilisation dans le traitement d'une tumeur, de préférence une tumeur métastatique.

7. La composition pharmaceutique orale selon l'une quelconque des revendications 2 à 5 pour utilisation dans l'inhibition d'une métastase de tumeur.

8. Utilisation du PAC-1 sous forme amorphe selon la revendication 1 dans la préparation d'une composition pharmaceutique orale.

9. L'utilisation selon la revendication 8, sachant que la composition pharmaceutique orale est une composition pour le traitement d'une tumeur.

10. L'utilisation selon la revendication 8, sachant que la composition pharmaceutique orale est une composition pour le traitement d'une tumeur métastatique.

11. L'utilisation selon la revendication 8, sachant que la composition pharmaceutique orale est une composition pour l'inhibition d'une métastase de tumeur.

12. Composé PAC-1 représenté par la formule (I) suivante pour utilisation dans le traitement d'une tumeur, de préférence une tumeur métastatique ou pour utilisation dans l'inhibition d'une métastase de tumeur, sachant que le composé est sous forme amorphe :

(I).

PAC-1

structure 5

ZZ

ZZ4

ZZ2

ZZ3

S-PAC-1

**Figure 1. Chemical structure of PAC-1 and the analogs thereof.**

PAC-1 crystal ×40
ZYS-1 ×40

Figure 2. Comparison of microscope images of PAC-1 needle crystal versus ZYS-1.

PAC-1 crystal×100 times
PAC-1 crystal×200 times
PAC-1 crystal×400 times
PAC-1 crystal
ZYS-1 ×100 times
ZYS-1×200 times
ZYS-1 ×400 times
ZYS-1

Figure 3. Comparison of scanning electron images of PAC-1 needle crystal versus ZYS-1.

A
Lin (Counts)
2-Theta - Scale
d=16.56631
d=10.27952
d=8.99682
d=8.07971
d=7.61120
d=5.71807
d=5.260
d=5.05823
d=4.76350
d=4.62056
d=4.42940
d=4.21175
d=4.09029
d=3.98871
d=3.91802
d=3.75163
d=3.51959
d=3.45296
d=3.35532
d=3.26312
d=3.22771
d=3.08443
d=2.93937
d=2.63928
d=2.61106
d=2.51026
d=2.44163
d=2.07120
d=2.04015

Figure 4A. X-ray diffraction pattern of PAC-1 needle crystal

Lin (Counts)
5000
4000
3000
2000
1000
0
3
10
20
30
40
2-Theta - Scale

Figure 4B. X-ray diffraction pattern of ZYS-1 powder

100
80
60
40
20
0
% Inhibition
*
*
0.1
0.5
1
5
10
50
[μM]
Raphides
Amorphous powder

Figure 5. Effect of ZYS-1 and PAC-1 needle crystal on HGC-27 cell proliferation

100
50
0
-50
% Inhibition
***
0.1
0.5
1
5
10
50
[μM]
Raphides
Amorphous powder

Figure 6. Effect of ZYS-1 and PAC-1 needle crystal on K562/A02 cell proliferation

40
30
20
10
0
% Inhibition
0.1
0.5
1
5
10
50
[μM]
Raphides
Amorphous powder

Figure 7. Effect of ZYS-1 and PAC-1 needle crystal on A549 cell proliferation

150
100
50
0
% Inhibition
0.1
0.5
1.0
5.0
10.0
50.0
HT1080 [μM]
Raphides
Amorphous powder

Figure 8. Effect of ZYS-1 and PAC-1 needle crystal on HT-1080 cell proliferation

A
Control
ZYS-1 2μM
ZYS-1 4μM
0 h
4 h
8 h
12 h
B
Control
2μM
4μM
Migration areas(mm²)
0.4
0.3
0.2
0.1
0.0
4h
8h
12h

Figure 9. Effect of different doses of ZYS-1 on two-dimensional migration of HGC-27 cells (cell scratch assay)

A
Control
2μM
4μM
8μM
HGC-27
HT-1080
B
Number
60
40
20
0
Control
2μM
4μM
8μM
HGC-27
HT-1080

Figure 10. Effects of different doses of ZYS-1 on three-dimensional migration of HGC-27 and HT-1080 cells (Transwell assay)

Figure 11. Effect of different doses of ZYS-1 on HT-1080 cell invasion (Transwell assay)

A
150
100
50
0
-50
%Inhibition
-1
0
1
2
LogC
B
ZYS-1 2μM
Endostar 2.5μg/mL
Control
C
20
15
10
5
0
number of tubule
**
***
control
Endostar 2.5mg/ml
ZYS-1 2μM

Figure 12. Effect of ZYS-1 on HUVEC cell proliferation and tubule formation. A: Effect of ZYS-1 on HUVEC cell proliferation; B, C: Effect of ZYS-1 on tubular formation.

Control
ZYS-1 4μM
ZYS-1 8μM
Caspase-3
NF-κB
VEGF
XIAP
1
2
3
4
5
6
7
8
9
10
11
12

Figure 13. Effect of different doses of ZYS-1 on Caspase-3, NF-κB, VEGF and IXAP expression.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 101184491 A
- US 20070049602 A1
- WO 2010091382 A1


### Non-patent literature cited in the description


- **QUINN P et al.** *J Mol Biol,* 2009, vol. 388 (1), 144-158
- **PETERSON QP et al.** *J Med Chem,* 2009, vol. 52 (18), 5721-5731
- **ROY S et al.** *Proc Natl Acad Sci USA,* 2001, vol. 98 (11), 6132-6137
- **WEST DC et al.** *Mol Pharm.,* 2012, vol. 9 (5), 1425-1434
- **Q P. PETERSON ; D. C. HSU ; C J. NOVOTNY et al.** Discovery and Canine Preclinical Assessment of a Nontoxic Procaspase-3-Activating Compound. *Cancer Res,* 2010, vol. 70, 7232-7241
- **QUINN P. PETERSON et al.** *J. Med. Chem.,* 2009, vol. 52, 5721-5731